# EUROPEAN PATENT SPECIFICATION

(11) **EP 1 951 350 B1**
(45) Date of publication and mention of the grant of the patent: **07.05.2014**
(21) Application number: 06827811.8
(22) Date of filing: 14.11.2006
(51) Int. Cl.: A61M 25/01, A61M 25/09

(54) **ARTICULATING STEERABLE WIRE GUIDE**
GELENKIGE STEUERBARE DRAHTFÜHRUNG
GUIDE-FIL ARTICULÉ ORIENTABLE

(30) Priority: 21.11.2005 US 738760 P
(43) Date of publication of application: 06.08.2008
(73) Proprietor: Cook Medical Technologies LLC, Bloomington, IN 47404 (US)
(72) Inventor: AYALA, Juan Carlos, Penalolén, Santiago (CL); CARTER, Matthew, P., Dobson, North Carolina 27017 (US); HARDIN, David, M., Winston-Salem, North Carolina 27106 (US)
(74) Representative: Garratt, Peter Douglas
(86) International application number: PCT/US2006/044243
(87) International publication number: WO 2007/061702

(56) References cited:
- EP-A- 1 532 999
- WO-A-97/31677
- WO-A-2004/089456
- WO-A-2006/039216
- US-A- 5 037 391
- US-A- 5 522 819
- US-A1- 2002 032 455

## Description

### RELATED APPLICATIONS

The priority is claimed of U.S. Provisional Application Serial No. 60/738,760, filed November 21, 2005, which is a continuation-in-part of Non-Provisional Application Serial No. 11/234,990, filed September 26, 2005, which claims priority to U.S. Provisional Application Serial No. 60/614,908, filed on September 30, 2004. US 60/738,760 was converted to non-provisional Application 11/599,522, which was granted as US 8,070,693 on 6 December 2011.

### BACKGROUND

### 1. Field of Invention

This invention relates generally to devices for use in medical procedures, and more particularly, relates to steerable wire guides used separately or in conjunction with catheters or endoscopes. Specifically, this invention relates to an improved steerable wire guide including interlocking movable component access to hard to reach internal anatomy of a patient.

### 2. Related technology

Wire guides are used in various medical procedures involving the gastrointestinal system, including the pancreatobiliary system (i.e., the biliary tree), the stomach, and the esophagus. Wire guides can be long, slender, relatively flexible wires used to access a patient's narrow passageway during minimally invasive medical procedures. Wire guides can be cumbersome as well as requiring constant, delicate manipulation by the treating physician because of the length of the wire guide.

Alternately, wire guides can also be described as elongated flexible members used to provide a path along which another medical device can be moved. The path provided by the wire guide can be used to navigate an alternate medical device, such as a catheter, through a body vessel. In this configuration, the wire guide can provide an established path for inserting other devices, eliminating the need for performing delicate navigation procedures for each device passed through the vessel. The use of wire guides to define such a path is known in the art.

Wire guides must have the ability to be maintained in a stationary position during various medical procedures. In operation, the wire guide is navigated through a body vessel to the desired target location. Once positioned within the body vessel, a second medical device, frequently a cannula such as a catheter can be placed over the wire guide and transported to the desired target location for treatment.

A number of steerable medical devices are known in the art. Catheters that are themselves steerable are known from, for example, EP 1 532 999, which discloses an electrode catheter comprising an elongated tubular catheter body with a flexible tip section. A pair of slidably mated puller wires fixedly attached to each other at their distal ends extend from a handle at the proximal end of the catheter body through a lumen in the catheter body and into a lumen in the tip section. Proximal movement of one puller wire relative to the other results in deflection of the tip section.
In addition, cardiac instruments having steerable or deflectable distal tips are known, for example from WO97/31677. This document discloses a steerable or deflectable distal tip of a cardiac instrument that is provided by creating a differential tension in a distal portion, causing the distal portion either to curve if originally straight, or to straighten if originally curved. The differential tension can be created by a pull or a push wire disposed in a longitudinal lumen of the instrument, which wire is attached at its distal end to the instrument. In another embodiment, the differential tension is created by thermoelectrically heated by metallic strip which is disposed within a lumen within the instrument in the form of a stylet. In another embodiment, the stylet is deformed by virtue of differential tension being applied between a pair of strips comprising the stylet.
Further, medical devices having actuable loops at their distal ends are known in the art. For example, US 5,522,819 discloses a device for retrieving foreign bodies, such as a vaso-occlusive coil or other articles, from within vessels such as those of the cardiovascular system. Two small snare coils are joined at their distal ends to form a loop and the proximal ends of the loops are attached to one or more elongated actuator members. The loop size is adjusted by manipulation of one or more of the proximal ends of the elongated actuator members. This retrieval device may be used to ensnare and either reposition or remove foreign bodies located within a vessel lumen.
In another example, US 2002/0032455 (now issued as US 6,620,179 on 16 September 2003) discloses a clot disrupting wire/catheter assembly that includes an annular sleeve and a core wire that is positioned within the annular sleeve. The assembly also includes a distal end wire that is attached to the annular sleeve and the core wire. The clot disrupting wire/catheter assembly may be deployed by pulling or pushing the core wire, so that the distal wire element forms a loop and has an open expanded conformation.
In the case of a wire guide, the operator of the wire guide must navigate the wire guide through the body vessel. Often, the body vessel forms a torturous path as a result of natural bends or curves in the body vessel or in the alternative, unnatural impediments, such as tumors or build-ups, which may also exist. The existence of this torturous path makes the navigation of the wire guide difficult. For example, the presence of an impediment may block the wire guide from navigating further into the vessel to reach the target or repair location.

As a result of the complexity of the above-described procedures, physicians often need the assistance of another person to secure the wire guide in addition to any additional medical devices used. Consequently, the physician's assistant must divert his or her attention from his or her primary responsibilities such as checking the patient's vital signs, checking monitors for relevant information and carrying out other tasks to assisting with maintaining the stability of the steerable wire guide.

The related art includes several examples of wire guides having a straight flexible tip and an elongated body portion intended to aid in the navigation of the wire guide. The presence of the straight flexible tip, however, may in fact make navigation more difficult. For example, upon encountering an impediment, the straight flexible tip may bend toward one of the vessel walls. Further, the straight flexible tip may bend and tum back upon itself upon encountering the impediment. As a consequence the straight flexible tip may encounter a sudden sharp tum which makes further navigation difficult.

Examples of successful devices that have been developed to address this need in the art are disclosed in pending U.S. Application Serial No. 10/719,764, filed November 21,2003 (now issued as US 7,520,881 on 21 April 2009), and entitled "Loop Tip Wire Guide," which claims priority to U.S. Provisional Application Serial No. 60/430,466, filed on December 2, 2002; and in U.S. Application Serial No. 11/234,990, filed September 26, 2005, and entitled "Steerable Loop Tip Wire Guide," which claims priority to U.S. Provisional Application Serial No. 60/614,908 filed on September 30, 2004.

In the first application, a resilient loop and a closure member are affixed to the distal end of a wire guide. When this device is navigated through a body vessel and encounters an impediment, the distal end of the wire guide does not move relative to the remainder of the wire guide due to the presence of the loop and closure member. Instead, the loop deforms in response to the impediment. The resiliency of the loop creates a force opposing the impediment and directs the loop away from the impediment. Therefore, the remainder of the wire guide following the path created by the loop tip enables the wire guide to navigate around the impediment and continues along the interior of the vessel.

In the latter mentioned application, a steerable wire guide is provided that can be formed with or without a loop. The wire guide further includes a closure member to close the loop. In this configuration, the loop is static and makes a soft loop instead of a pointed end. The wire guide can be easily manipulated once inside the body vessel cavity. The wire guide deforms in accordance with the internal path of the body vessel. Yet, additional improved embodiments of wire guides are desirable.

The general purpose of the present invention overcomes problems in the prior art by providing an improved articulating steerable wire guide having multiple configurations yet sufficiently steerable to provide greater control by the user and safety when deployed. In situations where the point of treatment may be located in a side branch or beyond the bifurcation of the main vessel, there is a need for a wire guide that can be shifted and, durable as to be easily manipulated through the tortuous path. For this reason, a wire guide would be desirable to provide the user with greater ability of control. It would also be desirable to provide a steerable wire guide that can be turned in various degrees and configurations to provide access to any structure without substitution of any of its components.

### BRIEF SUMMARY

These and other objects and advantages of this invention will become apparent to a person of ordinary skill in this art upon careful reading of the detailed description of this application including the drawings as presented herein. The present invention relates to an articulating steerable wire guide. The wire guide comprises an elongated composite structure having a longitudinal axis, comprising a first member and second member, wherein the first member and second member are adjacent to each other and in communication such that the first member and second member together form a substantially circular cross-section,
and wherein the composite structure defines a leading portion and a body portion. The term "substantially circular" cross-section, as used herein, includes oval or elliptical cross-sections. The leading portion may comprise a unitary tip and the body portion may comprise a rigid body, the body portion having a first section and a second section, the first section and the second section of the body portion being axially slidably movable relative to each other. Both the first section and the second section are connected to the unitary tip. The word "rigid" as used herein means rigid enough to allow axial movement through an endoscopic passageway without compromising structural integrity.

When the first section of the body portion retracts relative to the second section of the body portion and the second section of the body portion advances forward relative to the first section of the body portion, the leading portion is directed in a first direction at an angle relative to the a longitudinal axis of the elongated composite structure. When the first section of the body portion advances relative to the second section of the body portion, the leading portion is directed ill a second direction, opposite to the first direction and at an angle relative to the longitudinal axis of the elongated composite structure.

The invention is a steerable wire guide having a longitudinal axis, comprising a composite structure having a leading portion and a body portion, wherein the composite structure comprises a first member and a second member. The first and second members comprise interlocking components such that the first member is configured to be securely attached to the second member. The first member and second member are, however, axially movable relative to each other, such that concurrent movement of the first and second members in a first direction causes the first and second members to bend in unison in a first direction to advance the leading portion, while the concurrent movement of the first and second members in a second direction retracts the leading portion.

The leading portion comprises a substantially soft material and the body portion comprises a substantially rigid material. The cross-section of the composite structure has a shape configuration, the shape configuration comprising a first member and a second member in communication with each other.

The wire guide comprises a first guiding wire section, a wire loop section and a second guiding wire section, the wire component being folded back on itself to form a generally central wire loop section; and a tubular sheath surrounding the first guiding wire section and the second guiding wire section.

The steerable wire guide can have a radiopaque marker on the composite structure. Independently, the steerable wire guide can have a covering positioned over at least a portion of the composite structure, particularly if needed to hold members of the wire guide adjacent to each other.

### BRIEF DESCRIPTION OF THE DRAWINGS

These and other features and advantages of the present invention will be better understood by reference to the following detailed description when considered in conjunction with the accompanying drawings, wherein:
**Figure 1** is a side view of a steerable wire guide; which does not form part of the invention;
**Figure 2** is a top view of the steerable wire guide of **Fig. 1**, illustrating one example of first and second members of a steerable wire guide, the cross-section of which is shown in **Fig. 8**;
**Figure 3** is a top view of the steerable wire guide of **Fig. 1** **in** a first configuration, wherein the leading portion of the composite structure is directed in a first direction, and at an angle relative to the longitudinal axis of the elongated composite structure;
**Figure 4** is a top view of the steerable wire guide of **Fig.1** in a second configuration, wherein the leading portion of the composite structure is directed in a second direction, opposite the first direction, and at an angle relative to the longitudinal axis of the elongated composite structure;
**Figure 5** is a top view of the steerable wire guide of **Fig.1**, illustrating the first and second members of the steerable wire guide;
**Figure 6** is a top view of the steerable wire guide of **Fig. 1** in an alternate configuration;
**Figure 7** is a top view of the steerable wire guide of **Fig. 1** in an alternate configuration;
**Figure 8** is a cross-sectional view of one example of a steerable wire guide taken along line **A-A** of **Fig. 2**, and illustrating first and second members of the steerable wire guide, as well as a unitary sheath surrounding them;
**Figure 9** is a cross-sectional view of a second example of a steerable wire guide taken along line **B-B** of **Fig. 2****,** and illustrating first and second members of the steerable wire guide;
**Figure 10** is a cross-sectional view of one embodiment of a steerable wire guide taken along line **B-B** of **Fig. 2**, and illustrating first and second members of the steerable wire guide according to the claims;
**Figure 11** is a cross-sectional view of a fourth example of a steerable wire guide taken along line **B-B** of **Fig. 2**, and illustrating first and second members of the steerable wire guide;
**Figure 12** is a cross-sectional view of a fifth example of a steerable wire guide taken along line **B-B** of **Fig. 2**, and illustrating first and second members of the steerable wire guide;
**Figure 13** is a cross-sectional view of a sixth example of a steerable wire guide taken along line **B-B** of **Fig. 2**, and illustrating first and second members of the steerable wire guide;
**Figure 14** is a cross-sectional view of a seventh example of a steerable wire guide taken along line **B-B** of **Fig. 2**, and illustrating first and second members of the steerable wire guide;
**Figure 15** is a cross-sectional view of a eighth example of a steerable wire guide taken along line **B-B** of **Fig. 2**, and illustrating first and second members of the steerable wire guide;
**Figure 16** is a cross-sectional view of a ninth example of a steerable wire guide taken along line **B-B** of **Fig. 2**, and illustrating first and second members of the steerable wire guide;
**Figure 17** is a cross-sectional view of a tenth example of a steerable wire guide taken along line **B-B** of **Fig. 2**, and illustrating first and second members of the steerable wire guide;
**Figure 18** is a cross-sectional view of a eleventh example of a steerable wire guide taken along line **B-B** of **Fig. 2****,** and illustrating first and second members of the steerable wire guide;
**Figure 19** is cross-sectional view of a twelfth example of a steerable wire guide taken along line **B-B** of **Fig. 2**, and illustrating first and second members of the steerable wire guide;
**Figure 20** is a cross-sectional view of a thirteenth example of a steerable wire guide taken along line **B-B** of **Fig. 2**, and illustrating first, second and third members of the steerable wire guide;
**Figure 21** is a cross-sectional view of a fourteenth example of a steerable wire guide taken along line **B-B** of **Fig. 2**, and illustrating two parallel lumens disposed within a larger lumen in the steerable wire guide;
**Figure 22** is an illustration of a steerable wire guide comprising a coating;
**Figure 23** an illustration of a steerable wire guide comprising a coating over a portion of the wire guide;
**Figure 24** is a top view of a releasable connector detachably connecting a gripping portion of a removable handle and a tubular member proximal end according to one embodiment of the invention;
**Figure 25** is a top view of a releasable connector detachably connecting a gripping portion of a removable handle and a tubular member proximal end according to an alternate embodiment of the invention;
**Figure 26** is a top view of a releasable connector detachably connecting a gripping portion of a removable handle and a tubular member proximal end according to a second alternate embodiment of the invention;
**Figure 27** is a top view of a releasable connector detachably connecting a gripping portion of a removable handle and a tubular member proximal end according to a third alternate embodiment of the invention;
**Figure 28** is a top view of a steerable wire guide comprising a loop;
**Figure 29** is a cross-section of the example of **Fig. 28**, taken along line **29-29** of **Fig. 28**;
**Figure 30** is a top view of the steerable wire guide of **Fig. 28** illustrating the first and second members concurrently moving distally and leading portion advancing in the first direction; and
Figure 31 is a top view of a steerable wire guide comprising a loop as shown in **Figs. 28** and **30**, further illustrating a ground section ofa wall of the loop.

### DETAILED DESCRIPTION OF THE DRAWINGS AND PREFERRED EMBODIMENTS

Turning now to the figures, reference numbers are used to designate corresponding elements in the figures. Although the present invention has been described with reference to preferred embodiments, those skilled in the art will recognize that changes may be made in form and detail without departing from the spirit and scope of the invention. As such, it is intended that the following detailed description be regarded as illustrative rather than limiting and that it is the appended claims, including all equivalents thereof, which are intended to define the scope of the invention. The designations "top view" and "side view" are for orientation relative to each other only, as the device can be turned in any direction, which allows operation in any plane and access to virtually any structure.

The following paragraphs have been provided by way of general introduction, and are not intended to limit the scope of the following claims. The presently preferred embodiments, together with further advantages, will be best understood by reference to the following detailed description.

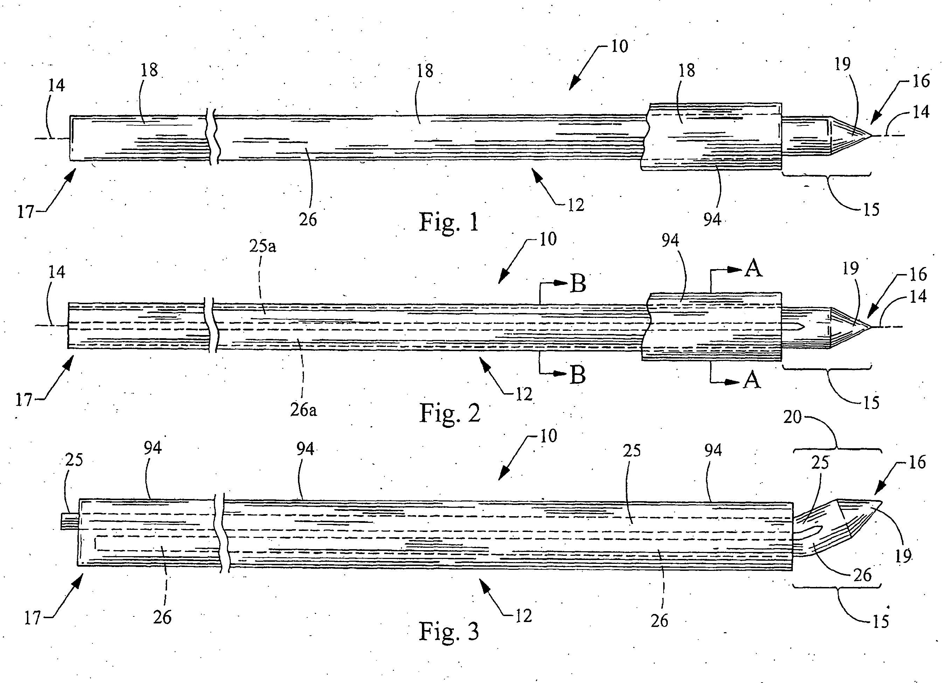

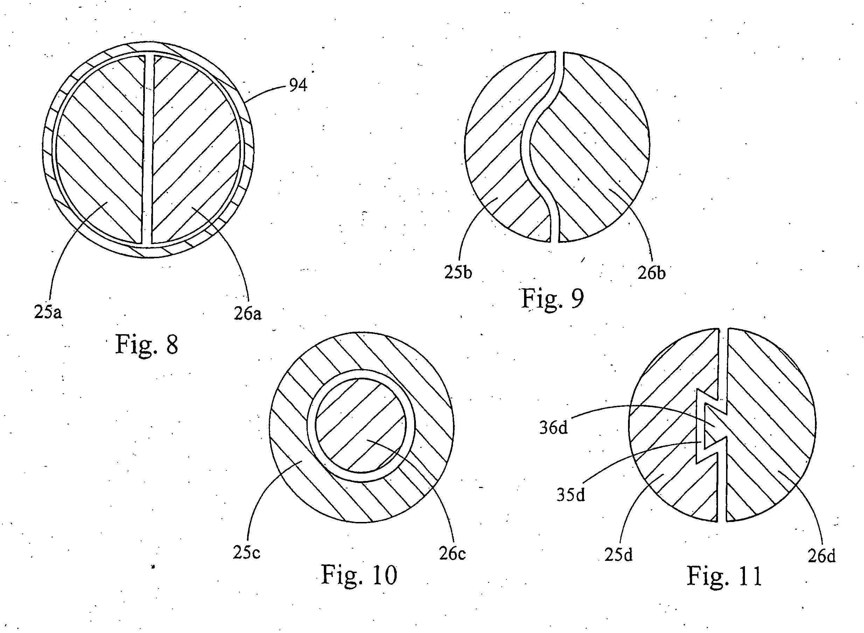

**Figure 1** illustrates a steerable wire guide **10**. Steerable wire guide **10** enables the user to direct and steer wire guide 10 through a body lumen. Wire guide **10 may** comprise a first member **25** (hidden from view in **Fig. 1**) and a second member **26** (such as first member **25a** and a second member **26a** shown in **Figs. 2** and **8**, or other pairs of first and second members whose cross-sections are shown **in** **Figs. 9-20**) interconnected to form an elongated composite structure **12.** The first member **25** and the second member **26** are joined such that the cross section of the composite structure **12** is substantially circular. The diameter of the substantially circular cross section may vary as desired. To maneuver the composite structure **12**, the first member **25** and the second member **26** slide relative to each other such that the advancement or extension of the first member **25** causes the second member **26** to retract. In the alternative, the retraction of the first member **25** causes the second member **26** to advance or extend, thereby allowing the user to control the direction in which the distal end **16** of the wire guide **10** extends. The first member **25** and the second member **26** can be connected together by a variety of methods known in the art.

The composite structure **12**, which is formed when the first member **25** and the second member **26** are joined, can be turned in any direction, preferably within 180 degrees of the longitudinal axis **14** of the composite structure **12**. The first member 25 and the second member **26** can also be oriented to slide in opposite directions such that the movement of the composite structure **12** can be turned 360 degrees.

As further shown in **Fig. 1**, the composite structure **12** has a leading portion **15** at the distal end **16** of wire guide **10** and a body portion **18** that includes the proximal end **17** of wire guide **10**. The leading portion **15** may have a taper wherein the diameter of the leading portion **15** is less than the diameter of the body portion **18**. When used with a catheter system, the leading portion **15** is inserted to correspond to the distal end of the catheter and the body portion **18** corresponds to the main body at the proximal region of the catheter. The body portion **18** can have a gradually increasing, gradually decreasing or uniform diameter. Preferably, the diameter of the body portion **18** should be sufficient to facilitate the transportation of medical devices over the composite structure **12** and may vary as desired.

The wire guide **10** has many advantages. The wire guide **10** can be maneuvered from the distal end **16** while the wire guide **10** is disposed within an internal body cavity. Consequently, delivery of medical devices or treatments to obstructed destination sites within a patient's body can be achieved. The wire guide **10** is flexible and can be used with or without the assistance of a catheter system. If the wire guide **10** is used with a catheter, the wire guide **10** can be used to manipulate the transport of the catheter or an alternative medical device through the patient's body cavities.

Any suitable material can be used for the composite structures **12**, and a variety of suitable materials are known to those skilled in the art. The material chosen need only be biocompatible and able to be formed into the structures described herein. Examples of suitable materials include stainless steel, shape memory material such as Nitinol or other nickel-titanium alloys, MP35N® and other nickel-cobalt alloys, Cobalt L-605™ and other cobalt-chromium alloys, other biocompatible metals, mental-alloys, as well as polymeric materials.

The interior surface of the wire guide **10** can be a solid wire or made from a material similarly suitable for acute use in the human body. The composite structure **12** can be made of the same material uniformly or from multiple materials having different inherent property characteristics.

The composite structure **12** comprises a tubular member forming a sheath **94** about first member **25** and second member **26**. The composite structure **12** canals be formed from a series of layers, or as a coated core structure. For example, the composite structure **12** can comprise a shape memory material with a solid core in one embodiment or a shape memory material core with a polytetrafluoroethylene covering in another embodiment. Depending on the desired range of movement of the wire guide, the appropriate material can be selected and configured as needed.

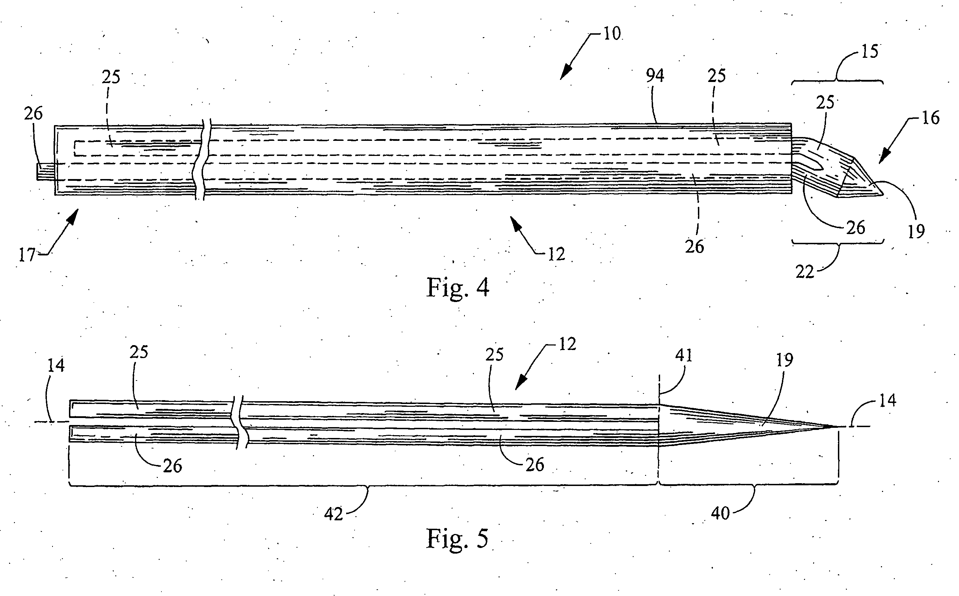

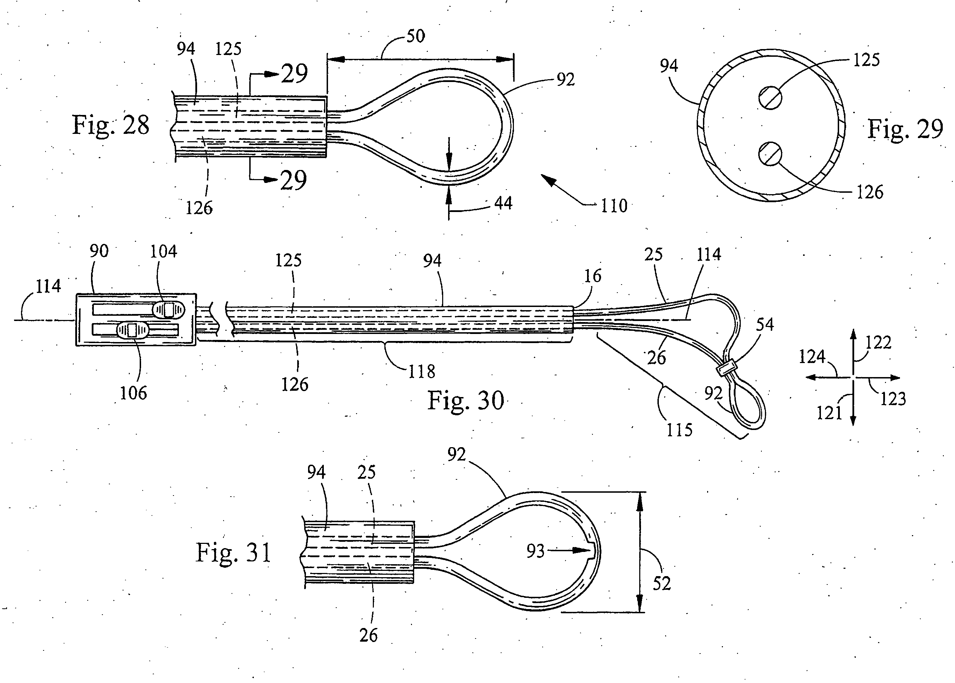

As shown in **Figs. 3** and **4**, respectively, the leading portion **15 of** structure **12** has a first configuration **20** and a second configuration **22**. It is contemplated that the leading portion **15** can have alternate configurations that permit advancement of the wire guide when deployed. The angle at which the leading portion **15** moves or bends is related to the material used and configuration of the first and second members **25, 26.** The leading portion **15** as shown in **Fig. 3** can be turned to be, for example, substantially perpendicular to the longitudinal axis **14** of composite structure **12.** The body portion **18** is substantially parallel to, or co-linear with, the longitudinal axis **14** during movement of the leading portion **15** from a first configuration **20** to a second configuration **22.** The leading portion **15** comprises a flexible loop **92,** as shown in **Figs. 28-31****.**An example of a wire guide with a loop tip that can be used in conjunction with the present invention is disclosed in the pending U.S. Application Serial No. 10/719,764, filed November 21, 2003, and entitled "Loop Tip Wire Guide".

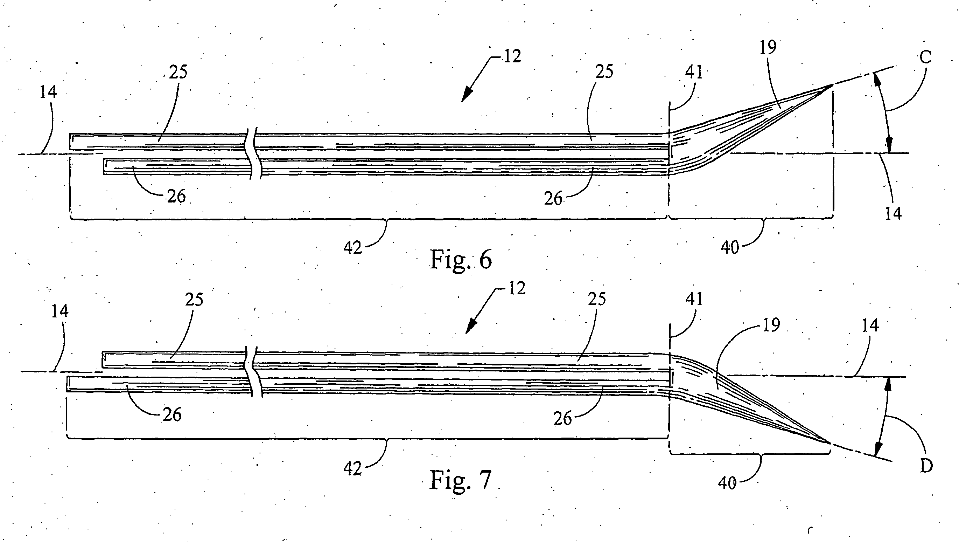

As shown in **Figs. 5-7****,** a junction **41** separates the soft body portion **40** and the rigid body portion **42.** In this example, which does not form part of the invention, members **25, 26 of** the composite structure **12** can be maneuvered at the distal end **16** of the composite structure **12** which in turn moves the tip **19** in one direction at an angle **C** (see **Fig. 6**) or in an opposite direction at an angle **D** (see **Fig. 7**) relative to the longitudinal axis **14**. The body portion **42** is composed of a rigid materials and the leading portion **40** is composed of a soft material, together forming the composite structure **12** of the steerable wire guide. In some embodiments, the rigid body portion **42** comprises a female section and a male section. The soft body portion **40** is a unitary structure. The soft body portion **40** can be maneuvered by sliding the first and second sections **25, 26** of the rigid body portion relative to each other, while the overall position of the rigid body portion does not change.

The first and second members **25, 26** are enclosed in a unitary sheath **94** (as shown in **Fig. 1**). The first and second members **25, 26** are joined at the distal end **16** of wire guide **10**, to allow for the movement of either member in a first or second direction.

Consequently, the first member 25 is turned and maneuvered forward, causing deflection of the wire guide 10. The joining techniques of the first and second members 25, 26 vary depending on the materials used.

In the configuration illustrated in Figs. 28-31, including a flexible loop 92 at the distal end 16, the wire guide is elongated and subsequently bent to form the loop. The thickness the wall 44 of the loop 92 should be sufficiently narrow to provide flexibility in either the lateral or longitudinal direction. The first and second members 25, 26 can slide in the lateral direction relative to each other maintaining a constant diameter through the wire guide. The thickness of the wall 44 of the loop 92 can be made smaller in one portion (for example, to help the loop collapse to fit into a catheter) by grinding down the wall in a desired section 93, as shown in Fig. 31.

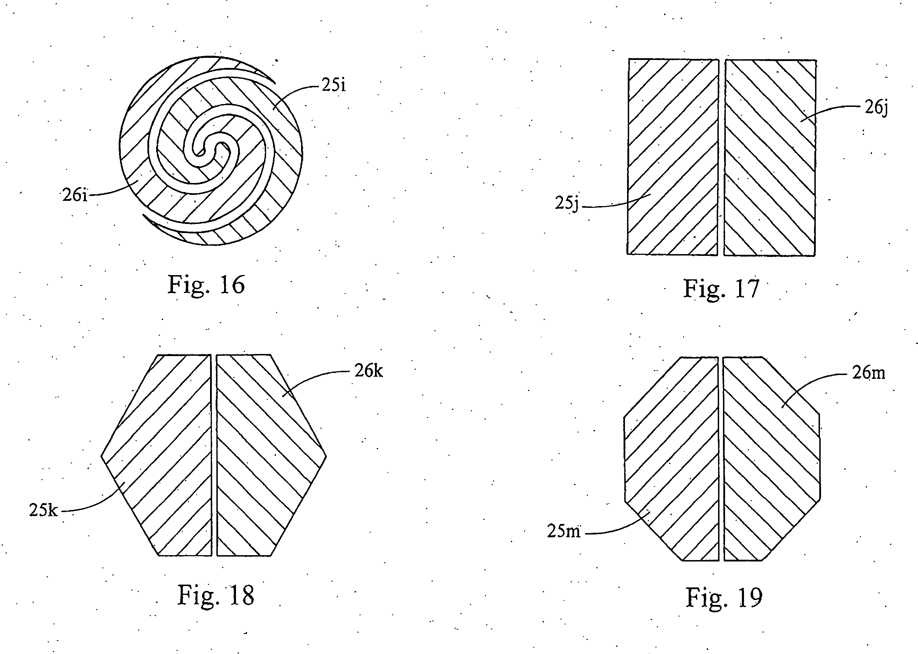

Alternate materials can be used. In one embodiment where a super elastic alloy is used, coil spring comprising platinum which can be easily viewed by x-ray, can be used for the loop or the elongated body or both. In yet other alternate embodiments, the cross section configuration of the loop and the first and second members can be anyone of rectangular, flat, triangular, trapezoidal, pentagonal or hexagonal. This is not exhaustive or all-inclusive. The wire guide 10 can be, for example, four-sided (see Fig. 17), six-sided (see Fig. 18) or eight-sided (see Fig. 19) to provide sufficient flexibility to the desired user.

Examples of first and second members which do not form part of the invention are illustrated in Figs. 8-20. In these figures, a variety of different cross-sectional configurations are shown. Some of the configurations feature interlocking female and male members. Figs. 8-20 are intended to be non-limiting examples and are used solely for providing a further understanding of the invention.

For example in Fig. 8, the first member 25a and the second member 26a are shaped as congruent halves. The combined cross section of first member 25a and second member 26a is substantially circular. The first member 25a and second member 26a are equally proportioned in size and shape. It is contemplated that the first member 25a and second member 26a can have a solid core, as shown in Fig. 8; or that either the first men. 1ber or the second member, or both, can have a hollow core.

In Fig. 9, an alternate shape for the first member 25 and second member 26 are shown as first member 25b and second member 26b, respectively.

In **Figs. 11****,** **12, 14, 15**, and **16**, the first members **25d, 25e, 25g, 25h** and 25i; and the second members **26d, 26e, 26g, 26h** and **26i**, respectively, are interconnected to form elongated composite structures. The combined cross section of the first member **25** and second member **26** is in each case substantially circular. The first member **25** and the second member **26** can be joined using various methods known in the art such that the first member **25** and second member **26** can be separated as needed. A coating (or covering) can be applied about the circumference of the first member **25** and second member **26** such that the union forms a composite structure.

In **Fig. 10****,** according to the claims, the first member **25c** is concentrically disposed around second member 26c. The first member **25c** and the second member **26c** are in communication with each other and can be assembled by inserting the second member 26c into the first member **25c.**

In **Fig. 11**, the first member **25d** has a channel **35d** that extends the length of the composite structure. The second member **26d** has a protruding portion **36d** that extends the length of the second member **26d**. The protruding portion **36d** of second member **26d** is inserted into the channel **35d** of first member **25d,** forming an elongated composite structure. Alternatively, both first member **25d** and second member **21d** can have both a channel and a protruding portion side-by-side, so that the protruding portion of each is disposed in the channel of the other.

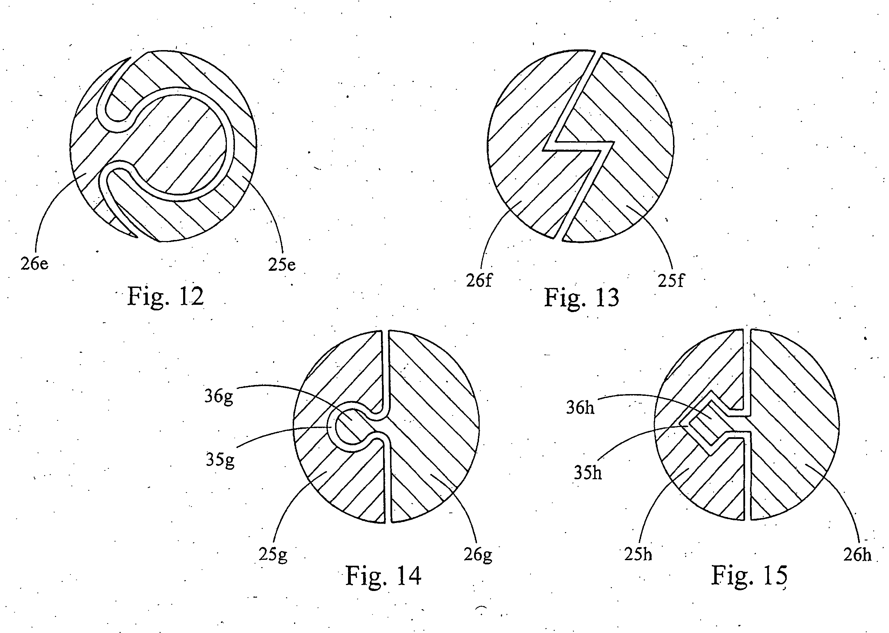

In **Fig.12**, the first / female member **25e** has an "U-shaped" configuration. The second / male member **26e** is inserted into the first member **25e**, forming a substantially circular cross section.

In **Fig. 13**, the first member **25f** has a first "zig-zag" configuration and the second member **26f** has a complementary "zigzag" configuration. The first member **25f** and second member **26f** are interconnected such that the first and second members **25f, 26f** together form a substantially circular cross section. The first member **25f** and the second member **26f** can be joined together using any method known in the art such that the first member **25f** and the second member **26f** form a composite structure that can be separated manually, using a mechanical device or a combination thereof.

In **Fig. 14**, the first / female member **25g** has a recessed portion **35g** and the second / male member **26g** has a protruding portion **36g**, such that the protruding portion **36g** is substantially disposed within the recessed portion **35g**.

In **Fig. 15**, the first / female member **25h** has a recessed portion **35h** having an alternate configuration, and the second member **26h** has a protruding portion **36h** such that first member **25h** and the second member **26h** together form a substantially circular cross section and elongated tubular body.

In **Fig. 16**, the first member **25i** has a first concentric configuration and the second member **26i** has a second concentric configuration, wherein the first configuration is in communication with the second configuration. In this embodiment, the first member **25i** and second member **26i** form a substantially elongated tubular structure.

In one embodiment (not shown), the steerable wire guide can be used to cannulate a duct. If the steerable wire guide is intended to cannulate the common bile duct, the suitable dimensions for the combined diameter of the first and second members can be about a range of 0.4 millimeters and 1 millimeter, and preferably, a diameter about 0.5 and 0.9 millimeters.

In **Fig.17**, the cross-sectional shape configuration has four sides. In this example, the wire guide **10** is comprised of a first member **25j** and a second member **26j** which each have a smaller four sided shape configuration.

In **Fig. 18**, the pentagonal cross sections of the first member **25k** and the second member **26k** together form a hexagon.

In **Fig. 19**, the hexagonal cross sections of the first member **25m** and the second member **26m** together form an octagon. In the configurations of **Figs. 18** and **19**, the cross section of the first and second members provides for the ability to have a larger diameter and a reinforced structural shape for withstanding compression forces in the body vessel.

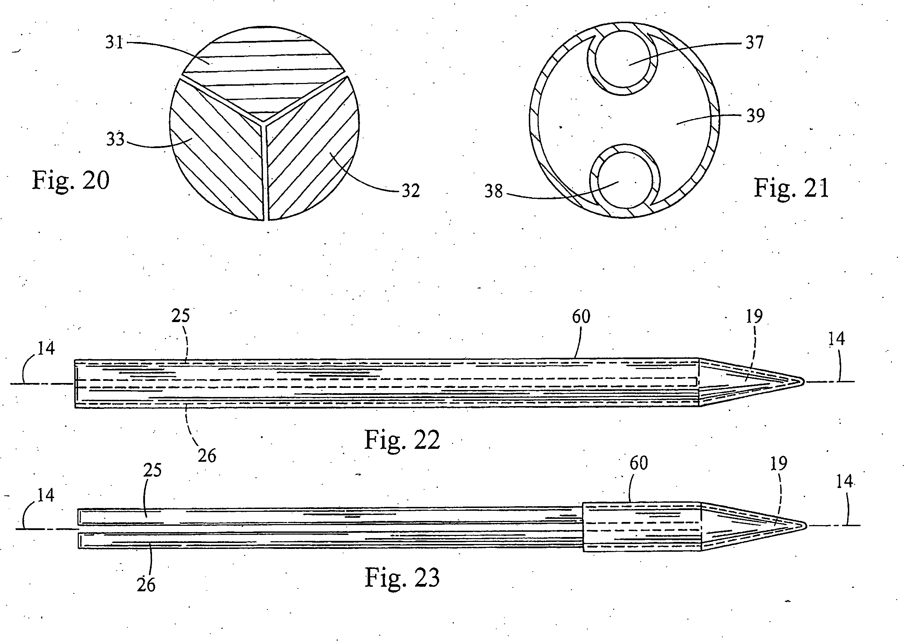

**Fig. 20** illustrates a cross section comprising tri-part members **31, 32** and **33**. The tri-part members can be equal shape and size or have varying configurations, such that in either case the total circumference formed is substantially circular

In **Fig. 21**, there are two parallel lumens **37, 38** disposed within a larger lumen **39**. In this configuration, multiple lumens are provided to accommodate multiple access points disposed through the wire guide.

The structures of the first member **25** and the second member **26** Can be formed by various techniques, depending on their shape. Complex shapes are best formed by extrusion. Flat surfaces can be cut, for example with a laser.

The first member **25** and the second member **26** are joined together at the distal end by a molded loop. The loop is overmolded or heat shrunk on the first and second members to join them together.

The steerable wire guide **10** can be used in conjunction with a catheter. The catheter can include a lumen extending therethrough whereby the steerable wire guide **10** can be inserted. Upon insertion, the steerable wire guide **10** can be controlled from the distal end **16** of the wire guide in the distal end of the catheter to maneuver the catheter in the patient's body cavity. An example of a catheter system that can be used in conjunction with the present invention is disclosed in the pending U.S. Application Serial No. 11/269,991, filed November 9, 2005, which claims priority to U.S. Provisional Application Serial No. 60/626,694, filed November 9, 2004, entitled "Steerable Catheter". The diameter of the steerable wire guide **10** can be significantly less than the diameter of the inner lumen of the catheter body. Despite the small diameter, the steerable wire guide **10** is well suited for injecting therapeutics or contrast agents or other treatments prescribed by a physician. The steerable wire guide **10** has sufficient torsional stability to facilitate steering of the steerable wire guide **10** within the lumen of the catheter.

Optionally, the steerable wire guide **10** can comprise a coating **60** as shown in **Fig. 22****.** Coating **60** can be positioned over the entire composite structure or just a portion thereof. Specifically, **Fig. 23** shows a partial coating of the composite structure **12** as well as the leading portion **15.** The coating **60** can be applied to retain the first and second members **25, 26.** The coating **60** can be positioned over a portion of, or the entire, composite structure **12**, including loop **92** (see **Figs. 28-31**).

The coating 60 can be polytetrafluoroethylene ("PTFE"), or another suitable material. Examples of suitable coverings include fluoropolymers, polyurethanes, and other suitable coatings used in the medical device arts. The coating 60 may be applied by dipping, molding, or spraying a suitable coating material, such as polytetrafluoroethylene, urethane, and/or other polymeric coatings directly to the desired portions of the steerable wire guide. Alternatively, the coating may be applied by heat shrinking a heat shrinkable material about the desired portions of the steerable wire guide.

One preferred coating comprises a thin PTFE heat shrinkable material. The heat shrinkable nature of these materials facilitates manufacturing while providing a lubricious coating, which facilitates navigation. In preferred embodiments, the thickness of the coating is between approximately 2.5 micrometers and 2.5 millimeters. In some embodiments, the thickness of the coating is between approximately 2.5 micrometers and 100 micrometers. In other embodiments, the thickness of the coating can be between approximately 2.5 micrometers and 50 micrometers. These preferred thicknesses provide suitable coatings while not adding significantly to the overall thickness of the device.

Radiopaque materials known in the art including, but not limited to, bismuth or gold can be added in the coating 60. Also, radiopaque markers known in the art can be placed on the composite structure 12. Several examples of suitable radiopaque materials and markers are known in the art, and any suitable material and/or marker can be utilized in the present invention.

The steerable wire guide 10, with or without coating 60, may be treated with a hydrophilic coating or hybrid polymer mixture, such as those based on polyvinyl pyrolidine in organic solvent solutions. These solutions make the wire guide particularly lubricious when in contact with body fluids, which aids in navigation.

A means for operating the steerable wire guide 10 such as a handle 90 is attached at the proximal end. The handle 90 allows the operator the ability to control the movement of the first and second members 25, 26 while simultaneously providing a structure to hold the steerable wire guide 10. The handle 90 can vary as needed and suitable configurations known in the art can be used. Optimally, the handle 90 includes a lumen extending therethrough wherein a wire can be disposed through each member and terminate at the proximal end **17**.

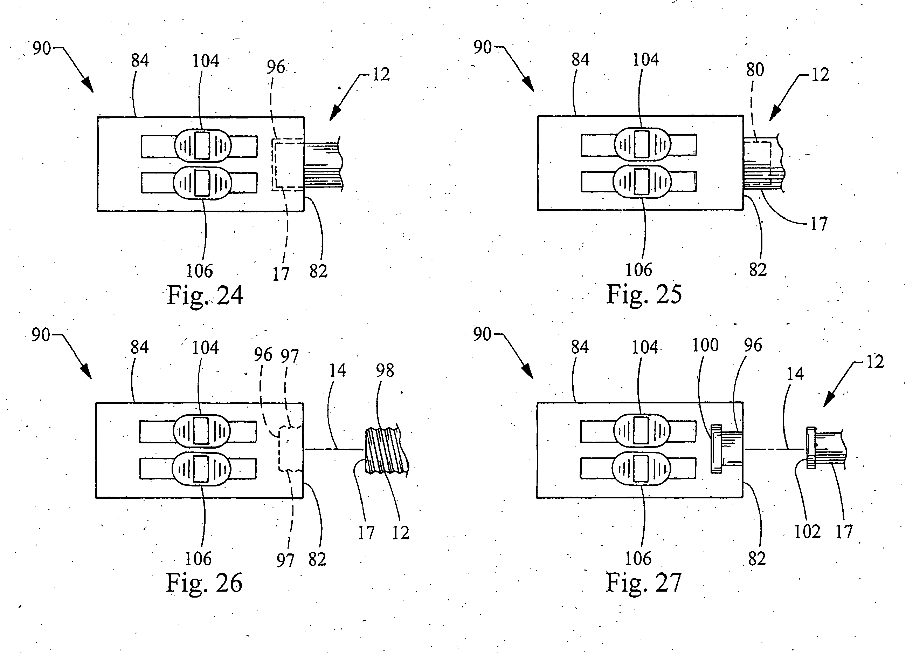

Optionally, as shown in **Figs. 24-27**, the handle **90** can be removable, detachably connected to the proximal end **17**. The handle **90** facilitates the user's ability to manipulate the first and second members **25, 26**. The handle **90** further provides the user with the option of retracting the composite structure **12** from the patient, thereby permitting the exchange of other medical devices over the composite structure **12**. Alternatively, the user is provided with the option of retracting the composite structure **12** from the patient, thereby permitting the exchange of other wire guides through the wire guide **10**.

The handle **90** comprises a gripping portion **84** and a releasable connector 82 that detachably interconnects the gripping portion **84** and the proximal end **17**. Several embodiments of releasable connector **82** are contemplated, including, but not limited to, an interference fit (see **Figs. 24** and **25**), a threaded connection (see **Fig. 26**) and a snap-fit connection (see **Fig. 27**). For example, **Fig. 24** and **25** illustrate exemplary embodiments of a releasable connector **82** forming an interference fit connection with the proximal end **17**. In the embodiment shown in **Fig. 24**, the releasable connector **82** comprises a longitudinal bore **96** with which the proximal end **17** forms an interference fit. Alternatively, in the embodiment shown in **Fig. 25**, the releasable connector **82** comprises a protrusion **80** defining an interior lumen through which the proximal portions of the first and second members **25, 26** extend. Protrusion **80** forms an interference fit at proximal end **17** of the composite structure **12**.

**Fig. 26** illustrates a releasable connector **82** having an internal longitudinal bore **96** having internal threads **97** which engage external threads **98** on the proximal end **17** of the composite structure **12.**

Alternatively, but not shown, the releasable connector comprises a protrusion having external threads and the proximal end comprises an expanded portion having internal threads. Thus, the removable handle is releasably attached to the proximal end by threading the protrusion of the releasable connector into the expanded portion of the proximal end of the wire guide.

Fig. 27 illustrates a non-limiting exemplary embodiment of a releasable connector 82 that forms a snap-fit connection with the proximal end 17. In this embodiment, the releasable connector 82 comprises a longitudinal bore 96 having an internal recess 100 and the proximal end 17 is received 'therein. The proximal end 17 comprises a ridge 102 that snaps into the internal recess 100 of the longitudinal bore 96.

In any of the configurations shown in Figs. 24-27, the first and second members 25 and 26 can be connected to control buttons 104 and 106, respectively, which can be moved forward or backward relative to handle 90, to advance or retract the member to which it is attached.

As shown in Figs. 28-31, the steerable wire guide 110 comprises an elongate member 112 having a longitudinal axis 114, a leading portion 115 and a body portion 118, the elongate member 112 further comprising a wire component 125, 92, 126 comprising a first guiding wire section 125, a wire loop section 92 and a second guiding wire section 126, the wire component being folded back on itself to form the wire loop section 92 in a generally central part of the wire component, and a tubular sheath 94 surrounding the first guiding wire section 125 and the second guiding wire section 126 to form the body portion 118 of the elongate member 112, and the wire loop section 92 of the wire component constituting the leading portion 115 of the elongate member 112; the first 125 and second 126 guiding wire sections being movable relative to each other and with respect to the tubular sheath 94 such that relative distal movement of the first guiding wire section 125 with respect to the second guiding wire section 126 directs the leading portion 115 in a first direction 121 at an angle relative to the longitudinal axis 114, relative distal movement of the second guiding wire section 126 with respect to the first guiding wire section 125 directs the leading portion 115 in a second direction 122 different from the first direction 121, concurrent distal movement of the first 125 and second 126 guiding wire sections moves the leading portion 115 in a third direction 123, and concurrent proximal movement of the first 125 and second 126 guiding wire sections moves the leading portion 115 in a fourth direction 124 opposite to the third direction 123; wherein the first direction 121 is away from a first side of the longitudinal axis 114 and the second direction 122 is away from a second side of the longitudinal axis 114, the first side generally being opposite the second side. Optionally, the steerable wire guide can further comprise a closure member **54** closing the wire loop section **92**, as shown in **Fig. 30**. Independently, a part of the wire loop section **92** can be ground smaller in one portion **93** of the wall **44** of the wire loop section **92**, as shown in Fig. **31**. If the wire used is 0.030 inches or 0.75 mm in diameter, for example, the wire can be ground to a thickness as small as 0.010 inches or 0.25 mm, in order to control the stiffness (and hence the loop size) of the wire loop section **92**. Alternatively, the wire need not be ground at all, if it is already of the desired stiffness to form the desired loop size.

A separate member defining a loop is affixed to two substantially straight wires to form the steerable wire guide of the present invention (not shown). This may be advantageous when it is desirable to form the loop and elongate member of different materials. For example, a nylon or silicone loop can be formed and attached, such as by a closure member, to an elongate member formed of Nitinol™.

Steerable wire guides **10** or **110** may be formed with or without closure member **54**. The closure member **54** can close the loop or wire loop section **92** such that no opening exists to the interior space of the loop or wire loop section **92**. Any suitable closure member can be used, including bonds, adhesives, and separate members. Examples of suitable closure members include sutures or other appropriate material tying the two sections together, adhesive bonds and other bonds (such as a solder bond, a welded bond, or a molded bond) and a connector (such as a rivet). The closure member **54** can be tightened, such as by crimping, to fix the loop or wire loop section **92** in overall size. In an alternate embodiment, not shown, the closure member **54** can be a molded bond. The loop or wire loop section **92** can be formed by molding two sections of the elongate member together. In another alternate embodiment, the closure member **54** is a welded bond. Two sections of the composite member **12** can be welded or soldered together to form loop or wire loop section **92**.

More specifically, the loop can be formed from two sections of the composite structure wound about each other. In yet another alternate embodiment (not shown), the closure member is integral with the composite member. In this case, the loop and the composite structure of the steerable wire guide are formed using laser cutting techniques as are known to those skilled in the art. The closure member 54 can be formed of any suitable material, and need only be biocompatible and capable of maintaining the loop or wire loop section 92 in a closed position. Preferably, the closure member 54 comprises a cannula formed of stainless steel or a shape memory material, such as Nitinol™. Also preferable, the closure member 54 is able to maintain a tightened position on the composite structure 12 upon application of a suitable force, such as by applying a crimping workload to the closure member 54.

The proximal end 17 may alternatively include a molded loop 92 to facilitate the steerable wire guide entry into catheters or other similar devices. The thickness of a wall 44 of molded loop 92 can be about 0.014 inches (0.36mm) and the width 52 of molded loop 92 can be about 0.075 inches (1.9mm). The length 50 of the molded loop 92 can vary as desired but preferably be about 0.150 inches (3.81mm). The molded loop 92 can be configured in alternate configurations as need. The preferred material of the molded loop is a plastic with a radiopaque coating, or a shape memory material. This is not an exhaustive material list. Further included in this configuration is a composite structure 12. The composite structure 12 can be comprised of a single or multiple wire configurations. The diameter of collapsed molded loop 92 should be approximately equally to the diameter of the composite structure 12.

Any other undisclosed or incidental details of the construction or composition of the various elements of the disclosed embodiment of the present invention are not believed to be critical to the achievement of the advantages of the present invention, so long as the elements possess the attributes needed for them to perform as disclosed. The selection of these and other details of construction were believed to be well within the ability of one rudimentary skilled in the area in view of the present disclosure. Illustrative embodiments of the present invention have been described in considerable detail for the purpose of disclosing a practical, operative structure whereby the invention may be practiced advantageously. The designs described herein are intended to be exemplary only.

It is therefore intended that the foregoing detailed description be regarded as illustrative rather than limiting, and that it is the following claims, that are intended to define the scope of this invention.

## Claims

1. A steerable wire guide having a longitudinal axis, comprising:
a composite structure comprising a first section (25c, 125) and a second section (26c, 126), said first and second sections being joined together by a loop (92) at the distal end of the wire guide, thus defining a leading portion at a distal end of the composite structure and an elongated body portion extending from the distal end, wherein, for at least a portion of which, the first section (25c, 125) is concentrically disposed around the second section (26c, 126) and the cross-sections of the first and second sections are substantially circular;
a handle attached at the proximal end (17) of the wire guide (10), to control movement of the first section (25c, 125) and the second section (26c, 126); and
a tubular sheath (94) surrounding the first section (25c, 125) and the second section (26c, 126);
wherein the first section (25c, 125) and second section (26c, 126) are movable relative to each other and with respect to the tubular sheath such that relative distal movement of the first section (25c, 125) with respect to the second section (26c, 126) directs the leading portion (115) in a first direction (121) at an angle relative to the longitudinal axis (114), relative distal movement of the second section (126) with respect to the first section (25c, 125) directs the leading portion (115) in a second direction (122) different from the first direction (121), concurrent distal movement of the first (25c, 125) and second (26c, 126) sections moves the leading portion (115) in a third direction (123), and concurrent proximal movement of the first (25c, 125) and second (26c, 126) sections moves the leading portion (115) in a fourth direction (124) opposite to the third direction (123); wherein the first direction (121) is away from a first side of the longitudinal axis (114) and the second direction (122) is away from a second side of the longitudinal axis (114), the first side generally being opposite the second side;
**characterized in that**
the loop (92) is plastic and is over-molded or heat shrunk on the first (25c, 125) and second (26c, 126) sections to join them together.

2. The steerable wire guide (10) of claim 1, wherein the thickness of a wall of the loop (92) is made smaller in one portion.

3. The steerable wire guide (10) of claim 1, comprising in addition a closure member (54), closing the loop (92) such that no opening exists to the interior space of the loop (92).

## Patentansprüche

1. Lenkbare Drahtführung mit einer Längsachse, umfassend:
eine Verbundstruktur, die einen ersten Abschnitt (25c, 125) und einen zweiten Abschnitt (26c, 126) umfasst, wobei der erste und der zweite Abschnitt über eine Schlaufe (92) am distalen Ende der Drahtführung miteinander verbunden sind und so einen vorderen Teil an einem distalen Ende der Verbundstruktur und einen sich vom distalen Ende erstreckenden länglichen Körperteil definieren, wobei der erste Abschnitt (25c, 125) zumindest zum Teil konzentrisch um den zweiten Abschnitt (26c, 126) herum angeordnet ist und die Querschnitte des ersten und des zweiten Abschnitts im Wesentlichen kreisförmig sind,
einen Griff, der am proximalen Ende (17) der Drahtführung (10) angebracht ist, um die Bewegung des ersten Abschnitts (25c, 125) und des zweiten Abschnitts (26c, 126) zu steuern, und
eine röhrenförmige Hülse (94), die den ersten Abschnitt (25c, 125) und den zweiten Abschnitt (26c, 126) umgibt,
wobei der erste Abschnitt (25c, 125) und der zweite Abschnitt (26c, 126) bezüglich einander und bezüglich der röhrenförmigen Hülse beweglich sind, so dass eine relative distale Bewegung des ersten Abschnitts (25c, 125) bezüglich des zweiten Abschnitts (26c, 126) den vorderen Teil (115) in einem Winkel zu der Längsachse (114) in eine erste Richtung (121) lenkt, eine relative distale Bewegung des zweiten Abschnitts (126) bezüglich des ersten Abschnitts (25c, 125) den vorderen Teil (115) in eine sich von der ersten Richtung (121) verschiedene zweite Richtung (122) lenkt, eine gleichzeitige distale Bewegung des ersten (25c, 125) und des zweiten Abschnitts (26c, 126) den vorderen Teil (115) in eine dritte Richtung (123) lenkt und eine gleichzeitige proximale Bewegung des ersten (25c, 125) und des zweiten Abschnitts (26c, 126) den vorderen Teil (115) in eine vierte Richtung (124) lenkt, die der dritten Richtung (123) entgegengesetzt ist, wobei die erste Richtung (121) von einer ersten Seite der Längsachse (114) weg verläuft und die zweite Richtung (122) von einer zweiten Seite der Längsachse (114) weg verläuft, wobei die erste Seite der zweiten Seite allgemein gegenüberliegt, **dadurch gekennzeichnet, dass**
die Schlaufe (92) Kunststoff ist und über den ersten (25c, 125) und den zweiten Abschnitt (26c, 126) gespritzt oder darauf aufgeschrumpft ist, um diese miteinander zu verbinden.

2. Lenkbare Drahtführung (10) nach Anspruch 1, wobei die Dicke einer Wand der Schlaufe (92) in einem Teil geringer ausgeführt ist.

3. Lenkbare Drahtführung (10) nach Anspruch 1, ferner umfassend ein Schließelement (54), das die Schlaufe (92) so schließt, dass keine Öffnung zum Innenraum der Schlaufe (92) vorliegt.

## Revendications

1. Fil-guide orientable ayant un axe longitudinal, comprenant :
une structure composite comprenant une première section (25c, 125) et une deuxième section (26c, 126), lesdites première et deuxième sections étant reliées l'une à l'autre par une boucle (92) à l'extrémité distale du fil-guide, définissant ainsi une partie avant à une extrémité distale de la structure composite et une partie de corps allongé s'étendant à partir de l'extrémité distale, pour au moins une partie de cette structure composite, la première section (25c, 125) étant disposée de manière concentrique autour de la deuxième section (26c, 126) et les sections transversales des première et deuxième sections étant sensiblement circulaires ;
une poignée fixée à l'extrémité proximale (17) du filguide (10), pour commander le déplacement de la première section (25c, 125) et de la deuxième section (26c, 126) ; et
une gaine tubulaire (94) entourant la première section (25c, 125) et la deuxième section (26c, 126) ;
la première section (25c, 125) et la deuxième section (26c, 126) étant déplaçables l'une par rapport à l'autre et par rapport à la gaine tubulaire de telle sorte qu'un déplacement distal relatif de la première section (25c, 125) par rapport à la deuxième section (26c, 126) dirige la partie avant (115) dans un premier sens (121) suivant un angle par rapport à l'axe longitudinal (114), qu'un déplacement distal relatif de la deuxième section (126) par rapport à la première section (25c, 125) dirige la partie avant (115) dans un deuxième sens (122) différent du premier sens (121), qu'un déplacement distal simultané de la première (25c, 125) et de la deuxième (26c, 126) section déplace la partie avant (115) dans un troisième sens (123), et qu'un déplacement proximal simultané de la première (25c, 125) et de la deuxième (26c, 126) section déplace la partie avant (115) dans un quatrième sens (124) opposé au troisième sens (123) ; le premier sens (121) étant à l'écart d'un premier côté de l'axe longitudinal (114) et le deuxième sens (122) étant à l'écart d'un deuxième côté de l'axe longitudinal (114), le premier côté étant généralement opposé au deuxième côté ;
**caractérisé en ce que**
la boucle (92) est plastique et est surmoulée ou thermorétractée sur la première (25c, 125) et la deuxième (26c, 126) section pour les relier l'une à l'autre.

2. Fil-guide orientable (10) selon la revendication 1, dans lequel l'épaisseur d'une paroi de la boucle (92) est plus petite dans une partie.

3. Fil-guide orientable (10) selon la revendication 1, comprenant en outre un organe de fermeture (54) fermant la boucle (92) de telle sorte qu'il n'existe pas d'ouverture sur l'espace intérieur de la boucle (92).
